# EUROPÄISCHE PATENTSCHRIFT

(11) **EP 3 399 311 B1**
(45) Veröffentlichungstag und Bekanntmachung des Hinweises auf die Patenterteilung: **26.02.2020**
(21) Anmeldenummer: 18169489.4
(22) Anmeldetag: 26.04.2018
(51) Int. Cl.: G01N 33/497, G01N 33/00

(54) **VORRICHTUNG ZUR VORBEHANDLUNG EINES ZU UNTERSUCHENDEN GASES**
DEVICE FOR PRETREATING A GAS TO BE TESTED
DISPOSITIF DE PRÉTRAITEMENT D'UN GAZ À ANALYSER

(30) Priorität: 03.05.2017 DE 102017207430
(43) Veröffentlichungstag der Anmeldung: 07.11.2018
(73) Patentinhaber: Robert Bosch GmbH, 70442 Stuttgart (DE)
(72) Erfinder: Thuersam, Markus, 71263 Weil Der Stadt (DE); Sahner, Kathy, 68167 Mannheim (DE); Jank, Heike, 71394 Kernen Im Remstal (DE); Schneider, Stefan, 71717 Beilstein (DE)

(56) Entgegenhaltungen:
- EP-A2- 2 168 480
- EP-A2- 2 862 514
- WO-A1-2010/115694
- DE-A1-102011 080 765
- US-A1- 2009 308 136

## Beschreibung

### Stand der Technik

Es ist bekannt, dass die Konzentration bestimmter Gase in der Atemluft eines Menschen auf Krankheiten hinweisen können. Unter Atemluft ist dabei die von dem Menschen ausgeatmete Luft zu verstehen. So kann beispielsweise eine erhöhte Konzentration von Stickstoffmonoxid (NO) auf Asthma bronchiale hinweisen. Dabei handelt es sich um eine chronische Erkrankung, bei der sich die Atemwege entzünden. Aufgrund einer Verengung der Atemwege kann die Entzündung zu anfallweiser Atemnot führen. Liegt eine Entzündung der Atemwege vor, wird mehr Stickstoffmonoxid (NO) von den Bronchien freigesetzt und in die Atemluft abgegeben als bei einem gesunden Menschen. Insbesondere zur Diagnose und/oder Behandlung von Asthma bronchiale ist es bekannt, die Atemluft eines Patienten zu untersuchen und insbesondere die Konzentration von Stickstoffmonoxid (NO) in der Atemluft zu messen. Dazu kann der Patient beispielsweise über ein Mundstück eines Messgeräts atmen, so dass die Atemluft über das Mundstück in das Messgerät gelangt. Dabei kann eine Vorbehandlung der zu untersuchenden Atemluft durch das Mundstück erfolgen. Bei bekannten Lösungen kann das Mundstück allerdings regelmäßig nur für einen Zweck, beispielsweise für die Messung der Konzentration eines bestimmten Gases, verwendet werden.

Artverwandte Vorrichtungen sind z.B. aus EP2862514 und US 2009/30813 bekannt.

### Offenbarung der Erfindung

Hier wird eine besonders vorteilhafte Vorrichtung zur Vorbehandlung eines zu untersuchenden Gases vorgestellt. Die abhängigen Ansprüche geben besonders vorteilhafte Weiterbildungen der Vorrichtung an.

Die beschriebene Vorrichtung kann insbesondere bei der Untersuchung von Gas und insbesondere bei der Untersuchung von Atemluft verwendet werden. Insbesondere wenn Atemluft untersucht werden soll, ist die Vorrichtung bevorzugt als ein Mundstück ausgeführt. Ein Patient kann in dem Fall über die Vorrichtung atmen, wobei die von ihm ausgeatmete Luft (also die Atemluft) zur Untersuchung in ein Messgerät geleitet werden kann. Die Untersuchung des Gases kann insbesondere die Bestimmung von Konzentrationen der Bestandteile des Gases umfassen. Bevorzugt umfasst die Untersuchung des Gases durch das Messgerät insbesondere die Messung einer Konzentration von Stickstoffmonoxid (NO) und/oder von Stickstoffdioxid (NO₂).

Insbesondere wenn die beschriebene Vorrichtung bei der Untersuchung von Atemluft eingesetzt wird, ist es bevorzugt, dass die beschriebene Vorrichtung ein Mundstück zum einmaligen Gebrauch ist. Um die Übertragung von Krankheiten zu vermeiden, wird also für jeden Patienten ein neues Mundstück benutzt.

Das zu untersuchende Gas kann vor der Untersuchung durch das Messgerät mit der beschriebenen Vorrichtung vorbehandelt werden. Dazu weist die beschriebene Vorrichtung mindestens zwei Behandlungskomponenten auf. Die Behandlungskomponenten sind bevorzugt derart ausgeführt und angeordnet, dass das zu untersuchende Gas bei Durchströmen der beschriebenen Vorrichtung durch die Behandlungskomponenten hindurchströmt. Strömt das zu untersuchende Gas durch eine Behandlungskomponente, wird das Gas je nach Ausführung der jeweiligen Behandlungskomponente beispielswiese getrocknet, und/oder gereinigt. Auch kann sich die Behandlung auf einzelne Bestandteile des zu untersuchenden Gases beschränken. Beispielsweise kann eine Behandlungskomponente bewirken, dass ein Bestandteil des zu untersuchenden Gases eine chemische Reaktion eingeht und dadurch umgewandelt wird. Eine Behandlungskomponente kann also insbesondere eine katalytische Wirkung haben.

Für verschiedene Untersuchungen an dem zu untersuchenden Gas können verschiedene Vorbehandlungen des zu untersuchenden Gases notwendig oder zumindest vorteilhaft sein. Die beschriebene Vorrichtung ist derart flexibel ausgeführt, dass die Vorrichtung eine Vorbehandlung auf verschiedene Arten, insbesondere unter Verwendung verschiedener der Behandlungskomponenten, ermöglichen kann. Dazu kann das zu untersuchende Gas entlang eines Strömungsweges durch die Vorrichtung geführt werden, wobei die mindestens zwei Behandlungskomponenten entlang des Strömungsweges angeordnet sind. Bei Durchströmen der Vorrichtung entlang des Strömungsweges durchströmt das zu untersuchende Gas bevorzugt die mindestens zwei Behandlungskomponenten.

Bevorzugt sind die Behandlungskomponenten derart ausgeführt und angeordnet, dass für jede Behandlungskomponente (insbesondere individuell) einstellbar ist, ob eine Behandlung des zu untersuchenden Gases bei Durchströmen der jeweiligen Behandlungskomponente erfolgen soll, oder ob das zu untersuchende Gas unbehandelt durch die Behandlungskomponente hindurchströmen soll. Diese Tatsache kann man auch damit beschreiben, dass die einzelnen Behandlungskomponenten eingeschaltet und ausgeschaltet werden, wobei eine Behandlung der zu untersuchenden Luft nur durch die eingeschalteten Behandlungskomponenten erfolgt, während das zu untersuchende Gas die ausgeschalteten Behandlungskomponenten unbehandelt durchströmen kann.

Die einzelnen Behandlungskomponenten weisen jeweils mindestens einen Behandlungsabschnitt, bevorzugt genau einen Behandlungsabschnitt, auf. Durchströmt das zu untersuchende Gas die Behandlungskomponenten in einem Behandlungsabschnitt, erfolgt die entsprechende Behandlung des zu untersuchenden Gases. Durchströmt das zu untersuchende Gas die Behandlungskomponenten hingegen außerhalb der Behandlungsabschnitte, erfolgt keine Behandlung des zu untersuchenden Gases durch diese Behandlungskomponente. Eine als ein Filter dienende Behandlungskomponente kann beispielsweise einen Behandlungsabschnitt aufweisen, in dem Filterpapier vorgesehen ist. Außerhalb des Behandlungsabschnitts ist dabei bevorzugt kein Filterpapier vorgesehen, sodass das zu untersuchende Gas hier ungefiltert (also unbehandelt) durch die Behandlungskomponente hindurch strömen kann. Außerhalb der Behandlungsabschnitte weisen die Behandlungskomponenten bevorzugt kein Material auf. Das bedeutet, dass die Behandlungskomponenten außerhalb der Behandlungsabschnitte vorzugsweise ein Loch beziehungsweise eine Aussparung aufweisen. Alternativ ist es bevorzugt, dass die Behandlungskomponenten außerhalb der Behandlungsabschnitte ein Material aufweisen, dass keine Behandlung des zu untersuchenden Gases bewirkt. Beispielsweise kann außerhalb der Behandlungsabschnitte ein Gitter und/oder ein Netz aus einem Material vorgesehen sein, das zur Stabilität der Behandlungskomponente beiträgt, das aber von dem zu untersuchenden Gas ohne Wechselwirkung durchströmt werden kann. Die Behandlungsabschnitte können als aktive Bereiche der Behandlungskomponenten bezeichnet werden, weil in den Behandlungsabschnitten eine Behandlung des zu untersuchenden Gases erfolgt.

Um einstellen zu können, ob die Behandlungskomponenten in den Behandlungsabschnitten oder außerhalb der Behandlungsabschnitte durchströmt werden, können die Behandlungskomponenten jeweils in eine erste Stellung und in eine zweite Stellung gebracht werden. Ist eine Behandlungskomponente in der ersten Stellung, verläuft der Strömungsweg durch einen Behandlungsabschnitt dieser Behandlungskomponente. Ist die Behandlungskomponente in der zweiten Stellung, verläuft der Strömungsweg durch keinen der Behandlungsabschnitte dieser Behandlungskomponente.

Zwischen der ersten und der zweiten Stellung kann bevorzugt durch Verschieben und/oder Verdrehen der jeweiligen Behandlungskomponente gewählt werden. Es ist möglich, dass eine Behandlungskomponente kontinuierlich über einen Verstellbereich verstellt werden kann. Beispielsweise kann der Verstellbereich bei einer drehbar verstellbaren Behandlungskomponente einen Winkelbereich von 360° [Grad] umfassen. Es ist bevorzugt, dass eine drehbar verstellbare Behandlungskomponente unbegrenzt, also auch über 360° [Grad] hinaus, verdreht werden kann. Alternativ können Anschläge vorgesehen sein, die eine drehbare Verstellung der Behandlungskomponente auf einen Winkelbereich von weniger als 360° [Grad] begrenzt.

Die erste Stellung und die zweite Stellung können jeweils als einzelne Positionen des Verstellbereichs oder aber auch als Teilbereiche des Verstellbereichs ausgeführt sein. Bevorzugt ist es, dass die erste Stellung und die zweite Stellung zusammengenommen den gesamten Verstellbereich überdecken, so dass sich die Behandlungskomponente nur in der ersten Stellung oder in der zweiten Stellung, nicht aber in einer weiteren Stellung befinden kann. Die Teilbereiche können dabei zusammenhängend oder auch nicht-zusammenhängend ausgeführt sein. Bei einer über einen Verstellbereich von 360° [Grad] verdrehbaren Behandlungs-komponente beispielswiese kann die erste Stellung einem Teilbereich von 300° entsprechen und die zweite Stellung einem Teilbereich von den übrigen 60°. In einem anderen Beispiel umfasst die erste Stellung zwei Teilbereiche von jeweils 120°, wobei zwischen diesen Teilbereichen jeweils ein Teilbereich von 60° für die zweite Stellung vorgesehen ist.

Der Strömungsweg ist bevorzugt unabhängig davon, welche der Behandlungskomponenten sich in der ersten Stellung und welche in der zweiten Stellung befinden. Das bedeutet, dass das zu untersuchende Gas bevorzugt immer den gleichen Weg durch die beschriebene Vorrichtung nimmt. Je nach Einstellung der einzelnen Behandlungskomponenten befinden sich dabei die Behandlungsabschnitte auf diesem Weg oder nicht. Die Behandlungsabschnitte können beispielsweise in den Strömungsweg hineingedreht oder herausgedreht werden, wodurch die entsprechende Behandlung eingeschaltet beziehungsweise ausgeschaltet werden kann.

Die beschriebene Vorrichtung kann zusätzlich zu den beschriebenen (verstellbaren) Behandlungskomponenten weitere Komponenten aufweisen, die von dem zu untersuchenden Gas passiert werden und die nicht ausgeschaltet werden können. Insbesondere ist bevorzugt eine Kondensfalle vorgesehen, die zum Kondensieren von Flüssigkeiten (insbesondere von Wasser) aus dem zu untersuchenden Gas eingerichtet ist und die vorzugsweise nicht ausgeschaltet werden kann.

In einer bevorzugten Ausführungsform der Vorrichtung sind die Behandlungskomponenten entlang des Strömungswegs nacheinander angeordnet.

Bevorzugt kann das zu untersuchende Gas nur über den Strömungsweg durch die beschriebene Vorrichtung strömen. Der Strömungsweg umfasst bevorzugt keine Abzweigungen oder Nebenströmungswege. Dadurch kann eine besonders einfache Konstruktion der Vorrichtung erreicht werden. Damit das zu untersuchende Gas bei einem derartig ausgeführten Strömungsweg alle Behandlungskomponenten durchströmen kann, sind die Behandlungskomponenten bevorzugt entlang des Strömungsweges hintereinander angeordnet.

In einer weiteren bevorzugten Ausführungsform der Vorrichtung sind die Behandlungskomponenten scheibenartig ausgeführt, wobei die Behandlungskomponenten durch Drehen um eine Scheibenachse in die erste Stellung und die zweite Stellung gebracht werden können.

Unter einer scheibenartigen Ausführung ist insbesondere zu verstehen, dass die Behandlungskomponenten zylinderförmig ausgeführt sind, wobei eine Zylinderhöhe klein ist im Vergleich zu einem Zylinderdurchmesser der Behandlungskomponenten. In dem Fall entspricht die Scheibenachse der Zylinderachse. Die Behandlungsabschnitte der Behandlungskomponenten sind insbesondere in dieser Ausführungsform bevorzugt als Winkelabschnitte ausgeführt. Der Strömungsweg verläuft vorzugsweise parallel zu der Scheibenachse und ist bevorzugt von der Scheibenachse beabstandet. Das bedeutet, dass das zu untersuchende Gas die Behandlungskomponenten vorzugsweise außermittig durchströmt. Dadurch kann der Strömungsweg in der ersten Stellung der Behandlungskomponenten durch den jeweiligen Behandlungsabschnitt verlaufen und in der zweiten Stellung der Behandlungskomponenten außerhalb der Behandlungsabschnitte verlaufen.

In einer bevorzugten Ausführungsform der Vorrichtung sind die Behandlungskomponenten entlang der Scheibenachse angeordnet.

Die Vorrichtung ist bevorzugt derart ausgeführt, dass die Behandlungskomponenten nicht vollständig mit dem zu untersuchenden Gas beaufschlagt werden, sondern dass das zu untersuchende Gas nur jeweils durch einen Teil der Behandlungskomponente hindurchtreten kann. Nur wenn ein Behandlungsabschnitt einer Behandlungskomponente in dem so gestalteten Strömungsweg angeordnet ist, erfolgt eine Behandlung durch diese Behandlungskomponente.

Dazu kann der Strömungsweg insbesondere mit einer oder mehreren Blenden beschränkt werden. Insbesondere dazu ist die weitere Ausführungsform der Vorrichtung bevorzugt, in der die Vorrichtung mindestens eine Blende umfasst.

Die Blenden können vor den Behandlungskomponenten, zwischen den einzelnen Behandlungskomponenten und nach den Behandlungskomponenten angeordnet sein.

Der Strömungsweg ist vorzugsweise derart begrenzt, dass das zu untersuchende Gas nur auf einen Teil der Behandlungskomponenten auftrifft. Damit kann das zu untersuchende Gas je nach Stellung der Behandlungskomponenten auf einen Behandlungsabschnitt der jeweiligen Behandlungskomponenten auftreffen oder nicht. Eine derartige Begrenzung des Strömungsweges ist insbesondere durch die mindestens eine Blende möglich. Insbesondere steht aufgrund der mindestens einen Blende vorzugsweise nicht der gesamte Querschnitt der Vorrichtung zur Durchströmung für das zu untersuchende Gas zu Verfügung. Es kann dementsprechend nur ein Teil des gesamten Querschnitts für die Durchströmung genutzt werden. Eine Blende zum Erreichen dieses Zwecks kann den einzelnen Behandlungskomponenten vor- und gegebenenfalls auch zwischengeschaltet werden. Aussparungen in Blenden können so gedreht bzw. ausgerichtet werden, dass gezielt Teile von Behandlungskomponenten mit Gas beaufschlagt werden.

In einer weiteren bevorzugten Ausführungsform der Vorrichtung werden die Behandlungskomponenten über eine Steuereinheit gesteuert.

Die Behandlungskomponenten sind bevorzugt jeweils mit einem Verstellelement verbunden, das zur Verstellung der Behandlungskomponente zwischen der ersten Stellung und der zweiten Stellung bestimmt und eingerichtet ist. Das Verstellelement kann beispielsweise ein Motor, insbesondere ein Elektromotor sein. Das Verstellelement ist bevorzugt an eine Steuereinheit angebunden, die das Verstellen der Behandlungskomponenten steuert. Die Steuereinheit kann innerhalb der Vorrichtung oder außerhalb der Vorrichtung angeordnet sein. Die Steuereinheit umfasst vorzugsweise eine Schnittstelle zur Kommunikation mit einem Computersystem und/oder mit einem Benutzer.

In einer weiteren bevorzugten Ausführungsform der Vorrichtung ist mindestens ein Behandlungsabschnitt ein Purgefilter.

Der Purgefilter ist insbesondere dazu eingerichtet, Stickstoffdioxid (NO₂) aus dem zu untersuchenden Gas herauszufiltern. Dazu kann der Purgefilter insbesondere Aktivkohle umfassen.

Das ist insbesondere in einer weiteren bevorzugten Ausführungsform der Vorrichtung der Fall, in der mindestens ein Behandlungsabschnitt ein Konverter zur Konvertierung von Stickstoffmonoxid (NO) zu Stickstoffdioxid (NO₂) ist.

Durchströmt das zu untersuchende Gas den Konverter, wird im zu untersuchenden Gas enthaltenes Stickstoffmonoxid (NO) vorzugsweise vollständig zu Stickstoffdioxid (NO₂) umgewandelt. Mit einer Kombination aus einem Purgefilter und einem Konverter, die insbesondere in dieser Reihenfolge von dem zu untersuchenden Gas durchströmt werden, kann die Konzentration von Stickstoffmonoxid (NO) im zu untersuchenden Gas besonders einfach gemessen werden. Dazu kann zunächst das im zu untersuchenden Gas enthaltene Stickstoffdioxid (NO₂) entfernt werden. Anschließend kann das im zu untersuchenden Gas enthaltene Stickstoffmonoxid (NO) durch den Konverter in Stickstoffdioxid (NO₂) umgewandelt werden. Weil das ursprünglich im zu untersuchenden Gas enthaltene Stickstoffdioxid (NO₂) durch den Purgefilter entfernt wurde, ergibt sich die nach Durchströmen des Purgefilters und des Konverters vorliegende Konzentration von Stickstoffdioxid (NO₂) ausschließlich aus der ursprünglichen Konzentration von Stickstoffmonoxid (NO) im zu untersuchenden Gas. Durch eine Messung der Konzentration von Stickstoffdioxid (NO₂) kann so auf die ursprünglich vorliegende Konzentration von Stickstoffmonoxids (NO) geschlossen werden. Insbesondere ist dazu kein Sensor für Stickstoffmonoxid (NO) nötig. Ein Sensor für Stickstoffdioxid (NO₂) kann ausreichen. Das ist deshalb vorteilhaft, weil ein Sensor für Stickstoffdioxid (NO₂) regelmäßig einfacher ausgeführt ist als ein Sensor für Stickstoffmonoxid (NO). Erfolgt eine derartige Messung der Konzentration von Stickstoffmonoxid (NO) für die Atemluft eines Patienten, kann auch von einer Messung von fraktioniertem, exhalierten Stickstoffmonoxid (NO) gesprochen werden. Eine solche wird regelmäßig als "FeNO-Messung" abgekürzt. Eine FeNO-Messung kann insbesondere bei der Diagnose und/oder Behandlung von Asthma bronchiale eingesetzt werden.

In einer weiteren bevorzugten Ausführungsform der Vorrichtung ist mindestens ein Behandlungsabschnitt eine Trocknungskomponente.

Insbesondere Atemluft kann feucht sein. Damit ist gemeint, dass die Atemluft gasförmiges Wasser enthält. Das Wasser kann eine Untersuchung der Atemluft verfälschen. Mit der Trocknungskomponente kann das zu untersuchende Gas in der beschriebenen Vorrichtung getrocknet werden.

Insbesondere ist es bevorzugt, dass die beschriebene Vorrichtung einen Purgefilter, einen Konverter und eine Trocknungskomponente als die Behandlungskomponenten aufweist, die insbesondere in der beschriebenen Reihenfolge von dem zu untersuchenden Gas durchströmt werden.

In einer weiteren bevorzugten Ausführungsform weist die Vorrichtung einen Anschluss auf, an welchen ein Messgerät zur Untersuchung des Gases angeschlossen werden kann.

Mit der beschriebenen Vorrichtung wird das zu untersuchende Gas bevorzugt vorbehandelt, bevor die Untersuchung des Gases erfolgt. Über den Anschluss kann das zu untersuchende Gas nach der Behandlung durch die beschriebene Vorrichtung dem Messgerät zugeführt werden.

Als ein weiterer Aspekt wird eine Messanordnung vorgestellt, die eine Vorrichtung wie beschrieben und ein Messgerät zur Bestimmung von Gaskonzentrationen in zu untersuchendem Gas umfasst, wobei das Messgerät derart an einen Anschluss der Vorrichtung angeschlossen ist, dass das zu untersuchende Gas über die Vorrichtung vorbehandelt und dem Messgerät zugeführt werden kann.

Die weiter vorne für die Vorrichtung beschriebenen besonderen Vorteile und Ausgestaltungsmerkmale sind auf die beschriebene Messanordnung anwendbar und übertragbar, und umgekehrt.

In einer bevorzugten Ausführungsform der Messanordnung weist das Messgerät eine Messkammer mit einem Sensor zur Messung von Stickstoffdioxid (NO₂) auf.

Wird die beschriebene Messanordnung beispielsweise zur Untersuchung von Atemluft eines Patienten verwendet, kann der Patient über die beschriebene Vorrichtung atmen, wobei die Atemluft als das zu untersuchende Gas in der beschriebenen Vorrichtung behandelt werden kann. Anschließend kann die Luft über den Anschluss der beschriebenen Vorrichtung und eine vorzugsweise vorgesehene Leitung in die Messkammer des Messgeräts gelangen. In der Messkammer des Messgeräts kann mit dem Sensor die Konzentration von Stickstoffdioxid (NO₂) in der Atemluft gemessen werden. Sind in der beschriebenen Vorrichtung beispielsweise ein Purgefilter, ein Konverter und eine Trocknungskomponente vorgesehen, und befinden sich diese drei Behandlungskomponenten jeweils in der ersten Stellung, so dass eine Behandlung der Atemluft durch alle drei Behandlungskomponenten erfolgt, kann von der Messung der Konzentration von Stickstoffdioxid (NO₂) in der Messkammer des Messgeräts auf die Konzentration von Stickstoffmonoxid (NO) in der Atemluft (vor der Behandlung in der beschriebenen Vorrichtung) geschlossen werden. Dabei handelt es sich insbesondere um die weiter vorne beschriebene FeNO-Messung.

Als ein weiterer Aspekt wird ein Mundstück zur Vorbehandlung von zu untersuchender Atemluft umfassend die beschriebene Vorrichtung vorgestellt. Die weiter vorne für die Vorrichtung und für die Messanordnung beschriebenen besonderen Vorteile und Ausgestaltungsmerkmale sind auf das beschriebene Mundstück anwendbar und übertragbar. Das mit der Vorrichtung zu untersuchende Gas ist hier die zu untersuchende Atemluft. Das Mundstück ist bevorzugt dazu bestimmt und eingerichtet, gemäß der beschriebenen Messanordnung an das Messgerät angebunden zu werden.

Das Mundstück weist bevorzugt einen Anschluss auf, über den das Mundstück mit dem Messgerät verbunden werden kann. An der Seite des Anschlusses weist das Mundstück bevorzugt in jeder Richtung quer zu dem Anschluss eine Ausdehnung zwischen 30 mm und 55 mm [Millimeter], bevorzugt zwischen 35 mm und 50 mm auf. Besonders bevorzugt weist das Mundstück an der Seite des Anschlusses einen ovalen Querschnitt auf, wobei die größte Ausdehnung des ovalen Querschnitts vorzugsweise zwischen 40 mm und 50 mm und die kleinste Ausdehnung des ovalen Querschnitts vorzugsweise zwischen 35 mm und 40 mm liegt. In Richtung des Strömungswegs weist das Mundstück bevorzugt eine Ausdehnung von 40 mm bis 60 mm auf.

Weitere Einzelheiten der Erfindung und ein Ausführungsbeispiel, auf welches die Erfindung jedoch nicht beschränkt ist, werden an Hand der Zeichnungen näher erläutert. Es zeigen:
- Fig. 1:: eine schematische Darstellung einer Messanordnung mit einer Vorrichtung zur Vorbehandlung eines Gases für eine Untersuchung des Gases und mit einem Messgerät zur Untersuchung des Gases,
- Fig. 2 bis 6:: fünf schematische Darstellungen von Behandlungskomponenten der Vorrichtung aus Fig. 1 in unterschiedlichen Stellungen, und
- Fig. 7:: eine schematische Darstellung eines Mundstücks mit der Vorrichtung aus den Fig. 1 bis 6.

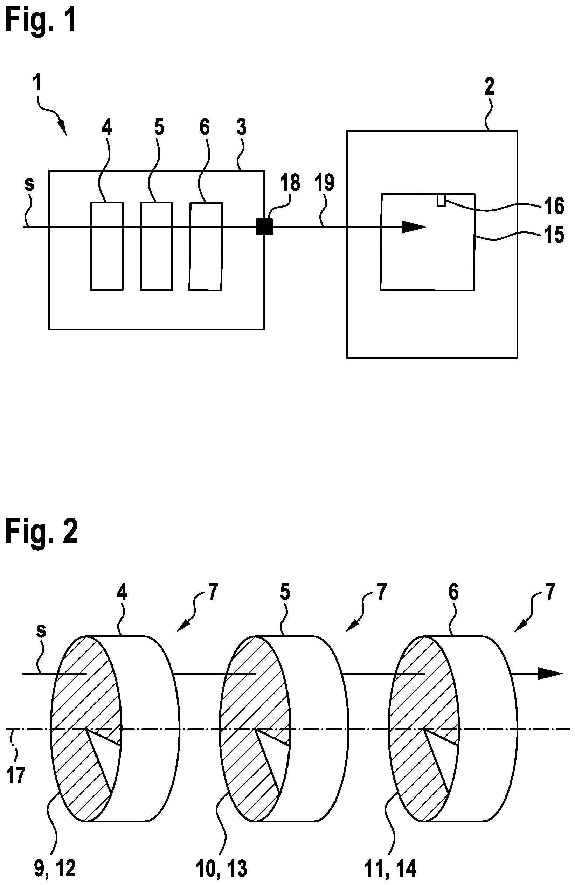

Fig. 1 zeigt eine Messanordnung 1 umfassend ein Messgerät 2, an welches eine Vorrichtung 3 zur Vorbehandlung eines zu untersuchenden Gases über einen Anschluss 18 und eine Leitung 19 angebunden ist. Entlang eines Strömungsweges s kann das zu untersuchende Gas durch die Vorrichtung 3 strömen, wobei drei Behandlungskomponenten (eine erste Behandlungskomponente 4, eine zweite Behandlungskomponente 5 und eine dritte Behandlungskomponente 6) durchströmt werden. Dabei kann eine Vorbehandlung des zu untersuchenden Gases erfolgen. Anschließend kann das zu untersuchende Gas in eine Messkammer 15 des Messgeräts 2 geleitet werden. In der Messkammer 2 ist ein Sensor 16 für die Konzentration von Stickstoffdioxid (NO₂) vorgesehen.

Die Fig. 2 bis 6 zeigen die Behandlungskomponenten 4, 5, 6 aus Fig. 1 in fünf verschiedenen Einstellungen. Zu erkennen ist, dass die Behandlungskomponenten 4, 5, 6 scheibenartig ausgeführt sind und entlang einer Scheibenachse 17 hintereinander angeordnet sind. Der Strömungsweg s verläuft parallel zu der Scheibenachse 17 durch die Behandlungskomponenten 4, 5, 6. Der Strömungsweg s ist von der Scheibenachse 17 beabstandet. Die erste Behandlungskomponente 4 umfasst einen ersten Behandlungsabschnitt 9, der als ein Purgefilter 12 ausgeführt ist. Die zweite Behandlungskomponente 5 umfasst einen zweiten Behandlungsabschnitt 10, der als ein Konverter 13 ausgeführt ist. Die dritte Behandlungskomponente 6 umfasst einen dritten Behandlungsabschnitt 11, der als eine Trocknungskomponente 14 ausgeführt ist.

Die Behandlungskomponenten 4, 5, 6 können jeweils in eine erste Stellung 7 und in eine zweite Stellung 8 gebracht werden. In der ersten Stellung 7 befindet sich der jeweilige Behandlungsabschnitt 9, 10, 11 der Behandlungskomponente 4, 5, 6 in dem Strömungsweg s. Dadurch kann eine Behandlung des zu untersuchenden Gases durch die Behandlungskomponenten 4, 5, 6 erfolgen. In der zweiten Stellung 8 befindet sich der jeweilige Behandlungsabschnitt 9, 10, 11 der Behandlungskomponenten 4, 5, 6 nicht in dem Strömungsweg s. Dadurch kann keine Behandlung des zu untersuchenden Gases durch die Behandlungskomponenten 4, 5, 6 erfolgen. Die in den Fig. 2 bis 6 gezeigten fünf verschiedenen Einstellungen unterscheiden sich durch die Stellung der Behandlungskomponenten 4, 5, 6.

In einer in Fig. 2 gezeigten ersten Einstellung befinden sich alle drei Behandlungskomponenten 4, 5, 6 in der ersten Stellung 7, sind also eingeschaltet. In der ersten Einstellung wird zunächst Stickstoffdioxid (NO₂) mit dem Purgefilter 12 aus dem zu untersuchenden Gas gefiltert. Anschließend wird Stickstoffmonoxid (NO) durch den Konverter 13 aus dem zu untersuchenden Gas in Stickstoffdioxid (NO₂) umgewandelt. Die ursprünglich im zu untersuchenden Gas vorliegende Konzentration von Stickstoffmonoxid (NO) ergibt sich damit aus der Konzentration von Stickstoffdioxid (NO₂), die nach Durchströmen des Purgefilters 12 und des Konverters 13 im zu untersuchenden Gas vorliegt. Anschließend wird das zu untersuchende Gas durch die Trocknungskomponente 14 getrocknet, so dass sich die erste Einstellung der Vorrichtung 3 insbesondere für die Untersuchung von (feuchter) Atemluft eignet. Insbesondere kann mit der ersten Einstellung eine Konzentration von Stickstoffmonoxid (NO) in der Atemluft durch Messung einer Konzentration von Stickstoffdioxid (NO₂) mit dem Sensor 16 bestimmt werden. Damit kann in der ersten Einstellung insbesondere eine FeNO-Messung durchgeführt werden. Dabei kann aus den vom Sensor 16 aufgenommenen Daten ein sogenannter FeNO-Wert, also die Konzentration von Stickstoffmonoxid (NO) in der Atemluft, berechnet werden. In der ersten Einstellung kann die Messanordnung 1 insbesondere zur Diagnose und/oder zur Behandlung von Asthma bronchiale verwendet werden.

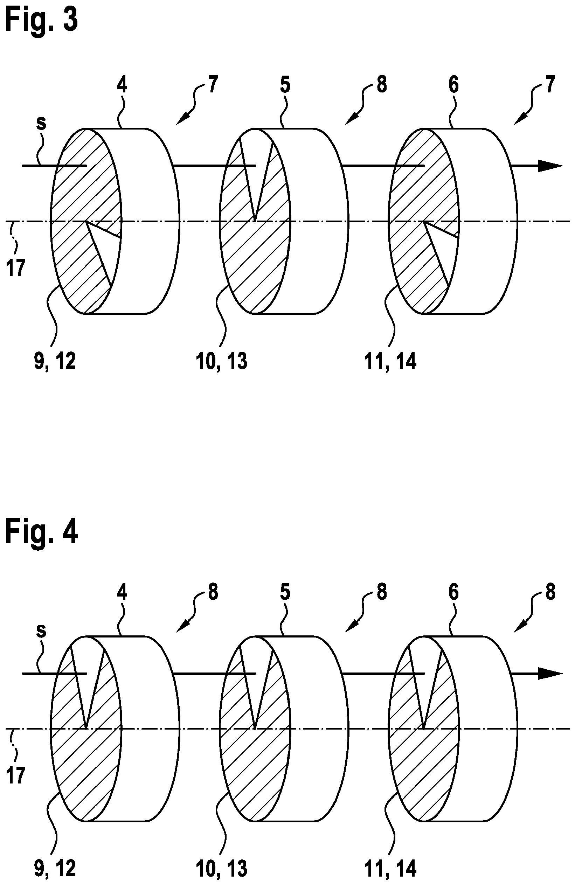

In einer in Fig. 3 gezeigten zweiten Einstellung befinden sich die erste Behandlungskomponente 4 und die dritte Behandlungskomponente 6 in der ersten Stellung 7, sind also eingeschaltet. Die zweite Behandlungskomponente 5 befindet sich in der zweiten Stellung 8, ist also ausgeschaltet. In der zweiten Einstellung kann zur Regeneration des Sensors 16 ein Spülen der Vorrichtung 3 durchgeführt werden. Beim Spülen können Stickstoffdioxid-Moleküle (NO₂-Moleküle), die sich am Sensor 16 abgesetzt haben, gelöst werden. Derartige Stickstoffdioxid-Moleküle (NO₂-Moleküle) könnten eine Messung verfälschen. Zum Spülen ist der Konverter 13 ausgeschaltet. Luft wird über die Vorrichtung 3 in die Messkammer 15 eingeleitet. Die Luft wird vorzugsweise der Umgebung der Vorrichtung 3 entnommen. Es handelt sich vorzugsweise insbesondere nicht um Atemluft eines Menschen. Aus der Luft wird Stickstoffdioxid (NO₂) mit dem Purgefilter 12 herausgefiltert. Anschließend wird die Luft mit der Trocknungskomponente 14 getrocknet. Die so erhaltene Luft enthält vorzugsweise kein Stickstoffdioxid (NO₂). Wird diese Luft über den Sensor 16 geleitet, insbesondere unter Erwärmung des Sensors 16 und/oder der Luft, können Stickstoffdioxid-Moleküle (NO₂-Moleküle) von dem Sensor 16 gelöst werden.

In einer in Fig. 4 gezeigten dritten Einstellung befinden sich alle drei Behandlungskomponenten 4, 5, 6 in der zweiten Stellung 8, sind also ausgeschaltet. Mit der dritten Einstellung kann insbesondere die Umgebungsluft analysiert werden und insbesondere eine Konzentration von Stickstoffdioxid (NO₂) in der Umgebungsluft gemessen werden. Dazu wird bevorzugt Umgebungsluft über die Vorrichtung 3 in die Messkammer 15 eingeleitet. Es findet keine Behandlung durch die Behandlungskomponenten 4, 5, 6 statt, so dass mit dem Sensor 16 eine Konzentration von Stickstoffdioxid (NO₂) in der Umgebungsluft unmittelbar gemessen werden kann. Diese kann bei einer Untersuchung beispielsweise von Atemluft als Vergleichswert verwendet werden. Weil die Umgebungsluft regelmäßig trocken und insbesondere trockener als Atemluft ist, wird die Trocknungskomponente 14 zur Untersuchung der Umgebungsluft nicht benötigt.

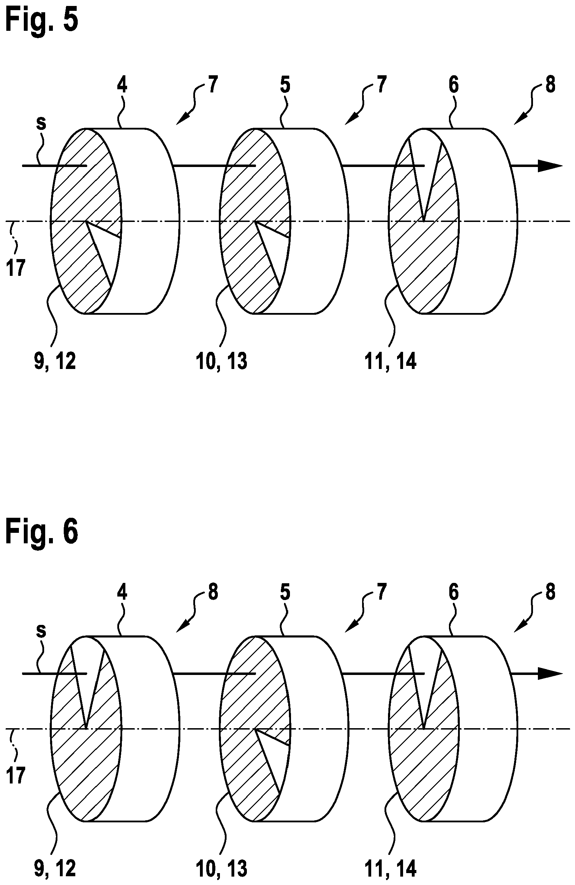

In einer in Fig. 5 gezeigten vierten Einstellung befinden sich die erste Behandlungskomponente 4 und die zweite Behandlungskomponente 5 in der ersten Stellung 7, sind also eingeschaltet. Die dritte Behandlungskomponente 6 befindet sich in der zweiten Stellung 8, ist also ausgeschaltet. Mit der vierten Einstellung kann insbesondere die Umgebungsluft analysiert werden und insbesondere eine Konzentration von Stickstoffmonoxid (NO) in der Umgebungsluft gemessen werden. Wie bei der ersten Einstellung kann auch in der vierten Einstellung die Konzentration von Stickstoffmonoxid (NO) durch eine Messung der Konzentration von Stickstoffdioxid (NO₂) erhalten werden. Im Unterschied zur ersten Einstellung ist in der vierten Einstellung die Trocknungskomponente 14 ausgeschaltet. Weil die Umgebungsluft regelmäßig trocken und insbesondere trockener als Atemluft ist, wird die Trocknungskomponente 14 zur Untersuchung der Umgebungsluft nicht benötigt.

In einer in Fig. 6 gezeigten fünften Einstellung befindet sich die zweite Behandlungskomponente 5 in der ersten Stellung 7, ist also eingeschaltet. Die erste Behandlungskomponente 4 und die dritte Behandlungskomponente 6 befinden sich in der zweiten Stellung 8, sind also ausgeschaltet. Mit der fünften Einstellung kann insbesondere die Umgebungsluft analysiert werden und insbesondere eine Summe der Konzentrationen von Stickstoffmonoxid (NO) und Stickstoffdioxid (NO₂) in der Umgebungsluft gemessen werden. In der fünften Einstellung kann Stickstoffmonoxid (NO) aus der Umgebungsluft mit dem Konverter 13 in Stickstoffdioxid (NO₂) umgewandelt werden. Weil der Purgefilter 12 ausgeschaltet ist, wird das bereits zuvor in der Umgebungsluft enthaltene Stickstoffdioxid (NO₂) nicht herausgefiltert, so dass aus dem mit dem Sensor 16 gemessenen Wert für die Konzentration von Stickstoffdioxid (NO₂) auf die Summe der Konzentrationen von Stickstoffmonoxid (NO) und Stickstoffdioxid (NO₂) in der Umgebungsluft geschlossen werden kann.

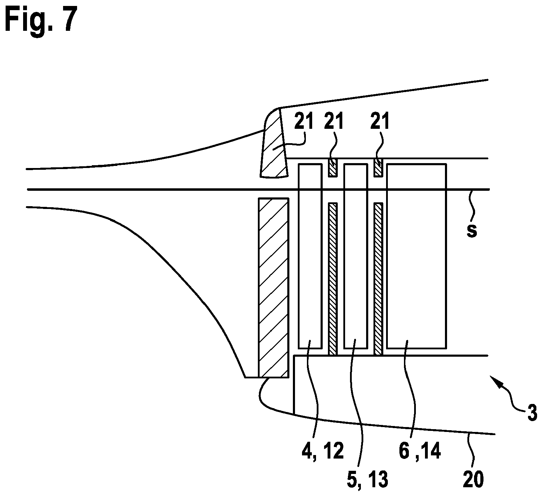

Fig. 7 zeigt ein Mundstück 20 umfassend die Vorrichtung 3 der Fig. 1 bis 6. Zu erkennen ist, dass der Strömungsweg s durch drei Blenden 21 beschränkt wird. Durch die Blenden 21 kann erreicht werden, dass der Strömungsweg s auf einen Teil der Behandlungskomponenten 4, 5,6 beschränkt ist.

## Patentansprüche

1. Vorrichtung (3) zur Vorbehandlung eines zu untersuchenden Gases, insbesondere ein Mundstück zur Vorbehandlung von zu untersuchender Atemluft, aufweisend einen Strömungsweg (s), entlang welchem das zu untersuchende Gas durch die Vorrichtung (3) führbar ist, umfassend mindestens zwei Behandlungskomponenten (4, 5, 6) zur Behandlung des zu untersuchenden Gases, aufweisend jeweils mindestens einen Behandlungsabschnitt (9, 10, 11), wobei jede der Behandlungskomponenten (4, 5, 6) wahlweise in eine erste Stellung (7) und in eine zweite Stellung (8) bringbar ist, wobei sich in der ersten Stellung (7) ein Behandlungsabschnitt (9, 10, 11) der Behandlungskomponenten (4, 5, 6) in dem Strömungsweg (s) befindet und eine Behandlung des zu untersuchenden Gases durch die Behandlungskomponenten (4, 5, 6) erfolgt, und wobei sich in der zweiten Stellung (8) kein Behandlungsabschnitt (9, 10, 11) der Behandlungskomponenten (4, 5, 6) in dem Strömungsweg (s) befindet und keine Behandlung des zu untersuchenden Gases durch die Behandlungskomponenten (4, 5, 6) erfolgt.

2. Vorrichtung (3) nach Anspruch 1, wobei die Behandlungskomponenten (4, 5, 6) entlang des Strömungswegs (s) nacheinander angeordnet sind.

3. Vorrichtung (3) nach einem der vorhergehenden Ansprüche, wobei die Behandlungskomponenten (4, 5, 6) scheibenartig ausgeführt sind, und wobei die Behandlungskomponenten (4, 5, 6) durch Drehen um eine Scheibenachse (17) in die erste Stellung (7) und die zweite Stellung (8) gebracht werden können.

4. Vorrichtung (3) nach einem der vorhergehenden Ansprüche , wobei mindestens ein Behandlungsabschnitt (9) ein Purgefilter (12) ist.

5. Vorrichtung (3) nach einem der vorhergehenden Ansprüche , wobei mindestens ein Behandlungsabschnitt (10) ein Konverter (13) zur Konvertierung von Stickstoffomonoxid (NO) zu Stickstoffdioxid (NO₂) ist.

6. Vorrichtung (3) nach einem der vorhergehenden Ansprüche , wobei mindestens ein Behandlungsabschnitt (11) eine Trocknungskomponente (14) ist.

7. Vorrichtung (3) nach einem der vorhergehenden Ansprüche, aufweisend einen Anschluss (18), an welchen ein Messgerät (2) zur Untersuchung des Gases angeschlossen werden kann.

8. Messanordnung (1) umfassend eine Vorrichtung (3) nach Anspruch 7 und ein Messgerät (2) zur Bestimmung von Gaskonzentrationen in zu untersuchendem Gas, wobei das Messgerät (2) derart an den Anschluss (18) der Vorrichtung (3) angeschlossen ist, dass das zu untersuchende Gas über die Vorrichtung (3) vorbehandelt und dem Messgerät (2) zugeführt werden kann.

9. Messanordnung (1) nach Anspruch 8, wobei das Messgerät (2) eine Messkammer (15) mit einem Sensor (16) zur Messung von Stickstoffdioxid (NO₂) aufweist.

10. Mundstück (20) zur Vorbehandlung von zu untersuchender Atemluft umfassend eine Vorrichtung nach einem der Ansprüche 1 bis 7.

## Claims

1. Device (3) for pretreating a gas to be analysed, in particular a mouthpiece for pretreating respiratory air to be analysed, having a flow path (s) along which the gas to be analysed is able to be conducted through the device (3), comprising at least two treatment components (4, 5, 6) for treating the gas to be analysed, which each have at least one treatment section (9, 10, 11), wherein each of the treatment components (4, 5, 6) is selectively able to be brought into a first position (7) and into a second position (8), wherein, in the first position (7), a treatment section (9, 10, 11) of the treatment components (4, 5, 6) is situated in the flow path (s) and the gas to be analysed is treated by the treatment components (4, 5, 6), and wherein, in the second position (8), no treatment section (9, 10, 11) of the treatment components (4, 5, 6) is situated in flow path (s) and the gas to be analysed is not treated by the treatment components (4, 5, 6) .

2. Device (3) according to Claim 1, wherein the treatment components (4, 5, 6) are arranged one after the other along the flow path (s).

3. Device (3) according to either of the preceding claims, wherein the treatment components (4, 5, 6) are of disc-like form, and wherein the treatment components (4, 5, 6) can be brought into the first position (7) and the second position (8) by rotation about a disc axis (17).

4. Device (3) according to one of the preceding claims, wherein at least one treatment section (9) is a purge filter (12).

5. Device (3) according to one of the preceding claims, wherein at least one treatment section (10) is a converter (13) for converting nitrogen monoxide (NO) into nitrogen dioxide (NO₂).

6. Device (3) according to one of the preceding claims, wherein at least one treatment section (11) is a drying component (14).

7. Device (3) according to one of the preceding claims, having a connection (18) to which a measurement appliance (2) for analysing the gas can be connected.

8. Measurement arrangement (1) comprising a device (3) according to Claim 7 and a measurement appliance (2) for determining gas concentrations in gas to be analysed, wherein the measurement appliance (2) is connected to the connection (18) of the device (3) such that the gas to be analysed can be pretreated via the device (3) and fed to the measurement appliance (2).

9. Measurement arrangement (1) according to Claim 8, wherein the measurement appliance (2) has a measurement chamber (15) with a sensor (16) for measuring nitrogen dioxide (NO₂).

10. Mouthpiece (20) for pretreating respiratory air to be analysed, comprising a device according to one of Claims 1 to 7.

## Revendications

1. Dispositif (3) pour le prétraitement d'un gaz à analyser, en particulier embout pour le prétraitement d'air respiratoire à analyser, présentant une voie d'écoulement (s) le long de laquelle le gaz à analyser peut être guidé à travers le dispositif (3), comprenant au moins deux composants de traitement (4, 5, 6) pour le traitement du gaz à analyser, présentant à chaque fois au moins une portion de traitement (9, 10, 11), chacun des composants de traitement (4, 5, 6) pouvant être amené de manière sélective dans une première position (7) et dans une deuxième position (8), dans la première position (7), une portion de traitement (9, 10, 11) des composants de traitement (4, 5, 6) se trouvant dans la voie d'écoulement (s) et un traitement du gaz à analyser étant effectué par les composants de traitement (4, 5, 6), et dans la deuxième position (8), aucune portion de traitement (9, 10, 11) des composants de traitement (4, 5, 6) ne se trouvant dans la voie d'écoulement (s) et aucun traitement du gaz à analyser n'étant effectué par les composants de traitement (4, 5, 6).

2. Dispositif (3) selon la revendication 1, dans lequel les composants de traitement (4, 5, 6) sont disposés les uns derrière les autres le long de la voie d'écoulement (s).

3. Dispositif (3) selon l'une quelconque des revendications précédentes, dans lequel les composants de traitement (4, 5, 6) sont réalisés en forme de disque et les composants de traitement (4, 5, 6) pouvant être amenés dans la première position (7) et dans la deuxième position (8) par rotation autour d'un axe du disque (17).

4. Dispositif (3) selon l'une quelconque des revendications précédentes, dans lequel au moins une portion de traitement (9) est un filtre de purge (12).

5. Dispositif (3) selon l'une quelconque des revendications précédentes, dans lequel au moins une portion de traitement (10) est un convertisseur (13) pour convertir du monoxyde d'azote (NO) en dioxyde d'azote (NO₂).

6. Dispositif (3) selon l'une quelconque des revendications précédentes, dans lequel au moins une portion de traitement (11) est un composant de séchage (14).

7. Dispositif (3) selon l'une quelconque des revendications précédentes, présentant un raccord (18) auquel peut être raccordé un appareil de mesure (2) pour analyser le gaz.

8. Agencement de mesure (1) comprenant un dispositif (3) selon la revendication 7 et un appareil de mesure (2) pour déterminer les concentrations de gaz dans le gaz à analyser, l'appareil de mesure (2) étant raccordé au raccord (18) du dispositif (3) de telle sorte que le gaz à analyser soit prétraité par le biais du dispositif (3) et puisse être acheminé à l'appareil de mesure (2).

9. Agencement de mesure (1) selon la revendication 8, dans lequel l'appareil de mesure (2) présente une chambre de mesure (15) avec un capteur (16) pour mesurer le dioxyde d'azote (NO₂).

10. Embout (20) pour le prétraitement d'air respiratoire à analyser, comprenant un dispositif selon l'une quelconque des revendications 1 à 7.
